# EUROPEAN PATENT SPECIFICATION

(11) **EP 0 575 462 B1**
(45) Date of publication and mention of the grant of the patent: **07.06.1995**
(21) Application number: 92907324.5
(22) Date of filing: 20.03.1992
(51) Int. Cl.: C12N 15/59, C12N 15/80

(54) **A PROCESS FOR PRODUCING CHYMOSIN**
HERSTELLUNGSVERFAHREN FÜR CHYMOSIN
PROCEDE DE PRODUCTION DE CHYMOSINE

(30) Priority: 22.03.1991 EP 91610020
(43) Date of publication of application: 29.12.1993
(73) Proprietor: GIST-BROCADES B.V., NL-2611 XT Delft (NL)
(72) Inventor: CHRISTENSEN, Tove, DK-2800 Lyngby (DK)
(86) International application number: DK9200089
(87) International publication number: WO9216633

(56) References cited:
- EP-A- 0 215 594
- EP-A- 0 238 023
- EP-A- 0 244 234
- Tibtech, Vol. 7, October 1989, GUNTER SAUNDERS et al.: "Heterologous geneexpression in filmentous fungi", see page 283 - page 287.

## Description

### FIELD OF INVENTION

The present invention relates to a process for the production of chymosin in filamentous fungi.

### BACKGROUND OF THE INVENTION

In recent years, procedures have been developed for the transformation of filamentous fungi, including Aspergillus niger and Aspergillus nidulans. US 4,885,249 (Allelix) describes a general process for the transformation of A. niger, exemplified by the introduction of plasmids carrying genes encoding selectable markers. EP 215 594 (Genencor) describes the expression and secretion of bovine chymosin in A. nidulans, using the signal sequence of A. niger glucoamylase to provide secretion. It appears from EP 215 594, however, that chymosin expressed in this system is only secreted at extremely low levels.

### SUMMARY OF THE INVENTION

It has surprisingly been found that when the gene encoding prochymosin is fused to the A. niger TAKA amylase signal sequence, the levels of chymosin secreted from A. niger are increased several fold compared to those apparent from EP 215 594.

Accordingly, the present invention relates to a process for the production of chymosin or a derivative thereof having same properties in filamentous fungi, the process comprising
(a) transforming a filamentous fungus host organism with a recombinant DNA vector which comprises a DNA sequence encoding prochymosin or a derivative thereof having same properties N-terminally fused to a DNA sequence encoding a signal peptide derived from the Aspergillus oryzae TAKA amylase gene or a derivative of said signal peptide having same properties,
(b) culturing the transformed filamentous fungus host organism in a suitable culture medium under conditions conducive to the expression of prochymosin and secretion thereof to the medium, and
(c) recovering the prochymosin or chymosin or derivative thereof from the medium.

In the present context, the term "derivative" is intended to indicate a polypeptide which is derived from the native chymosin or signal peptide (as the case may be) by suitably modifying the DNA sequence coding for the native chymosin/signal peptide, resulting in the addition of one or more amino acid at either or both the C- or N-terminal end, substitution of one or more amino acids at one or a number of different sites in the native amino acid sequence, deletion of one or more amino acids at either or both ends of the native amino acid sequence or at one or more sites within the native sequence, or insertion of one or more amino acids at one or more sites in the native amino acid sequence. Such modifications of the DNA sequence may be done by methods well known in the art.

The term "filamentous fungus" is intended to include the groups Phycomycetes, Zygomycetes, Ascomycetes, Basidiomycetes and fungi imperfecti, including Hyphomycetes such as the genera Aspergillus, Penicillium, Trichoderma, Fusarium and Humicola.

The presence of the signal sequence serves to direct the expressed prochymosin or prochymosin derivative effectively into the secretory pathway of the host cell so that prochymosin or chymosin may readily be isolated from the culture medium (at least some of the product recovered will be mature chymosin as the prochymosin secreted from the cells is either subjected to automaturation or maturation by proteases produced by the host cell). The amino acid sequence of the native TAKA-amylase signal peptide is as follows:
Met Met Val Ala Trp Trp Ser Leu Phe Leu Tyr Gly Leu Gln Val Ala Ala Pro Ala Leu Ala
It is, however, envisaged that derivatives (as defined above) of this sequence may also be employed for the present purpose.

The chymosin to be produced by the process of the invention is primarily bovine chymosin.

### DETAILED DESCRIPTION OF THE INVENTION

The vector may further comprise DNA sequences encoding functions facilitating gene expression, typically a promoter, transcription initiation sites, and transcription termination and polyadenylation functions.

The promoter which may be preceded by upstream activating sequences and enhancer sequences as known in the art may be any DNA sequence exhibiting a strong transcriptional activity in A. niger, and may be derived from a gene encoding an extracellular or intracellular protein such as an amylase, a glucoamylase, a protease, a lipase, a cellulase or a glycolytic enzyme.

Examples of suitable promoters are those derived from the gene encoding A. oryzae TAKA amylase, Rhizomucor miehei aspartlc proteinase, A. niger neutral α-amylase, A. niger acid stable α-amylase A. niger glucoamylase, Rhizomucor miehei lipase, or A. oryzae alkaline protease. Examples of promoters from genes encoding glycolytic enzymes are the A. oryzae triose phosphate isomerase, ADH and PGK promoters.

The filamentous fungus used as the host organism is preferably selected from an Aspergillus sp. such as A. niger or A. oryzae, in particular A. niger.

When the host organism is A. niger, a preferred promoter for use in the process of the present invention is the A. niger glucoamylase promoter or the A. oryzae TAKA amylase promoter as they exhibit a strong transcriptional activity in A. niger.

Termination and polyadenylation sequences may suitably be derived from the same sources as the promoter.

The techniques used to transform the host organism may suitably be adapted from the methods of transforming A. nidulans described in, for instance, Yelton et al., Proc. Natl. Acad. Sci. USA 81, 1984, pp. 1470-1474, or EP 215 594, or from the methods of transforming A. niger described in, for instance Buxton et al., Gene 37, 1985, pp. 207-215 or US 4,885,249. In the process of the present invention, A. niger may be transformed with a vector system comprising a DNA sequence coding for a selection marker which is capable of being incorporated in the genome of the host organism on transformation, but which is either not expressed by the host before transformation or not expressed in sufficient amounts to permit growth under selective conditions. Transformants can then be selected and isolated from non-transformants on the basis of the incorporated selection marker.

Suitable selection markers may be derived from the A. nidulans or A. niger argB gene, the A. nidulans trpC gene, the A. nidulans amdS gene, the Neurospora crassa pyr4 or DHFR genes, or the A. niger or A. oryzae niaD gene.

Preferred selection markers for use in the present invention are derived from the A. nidulans or A. niger amdS or argB genes. Wild-type A. niger strains are usually ArgB⁺ (which means that the argB gene is expressed in A. niger). Thus, if argB is chosen as the selection marker, an ArgB⁻ mutant strain of A. niger (which does not express the ArgB gene) must be used as the host organism. On the other hand, the amdS gene may be used as the selection marker in wild-type A. niger strains which do not express this gene in sufficient amounts to permit growth under selective conditions.

The gene coding for prochymosin fused to the TAKA-amylase signal sequence as well as to promoter and terminator sequences may be inserted in a vector containing the selection marker, or it may be inserted in a separate vector for introduction into the host cell. The vector or vectors may be linear or closed circular molecules. In one embodiment of the process of the invention, two vectors, one carrying the DNA sequence coding for the selection marker, and the other carrying the DNA sequence encoding prochymosin or a derivative thereof having same properties N-terminally fused to the DNA sequence encoding a signal peptide derived from the Aspergillus oryzae TAKA amylase gene, may be introduced into the host cell.

The medium used to culture the transformed host cells may be any conventional medium suitable for growing filamentous fungi. The transformants are usually stable and may be cultured in the absence of selection pressure. However, if the transformants are found to be unstable, the selection marker introduced into the cells may be used for selection.

The prochymosin or chymosin produced by the host cells may conveniently be recovered from the culture medium by well-known procedures including separating the cells from the medium by centrifugation or filtration, and precipitating proteinaceous components of the medium by means of a salt such as ammonium sulphate, followed by chromatographic procedures such as ion exchange chromatography, affinity chromatography, or the like.

The invention is further illustrated in the following examples which are not in any way to be construed as limiting to the scope of the invention as claimed.

### EXAMPLE 1

### Construction of a prochymosin expression vector

Unless otherwise indicated, the procedures used in the construction of the expression vector are standard procedures, e.g. as described in Sambrook, et al., Molecular Cloning: A Laboratory Manual, Cold Spring Harbor Laboratory, Cold Spring Harbor, NY, 1989.

Plasmid pToC51 (the construction of which is described in Example 12 of EP 238 023) carrying the 5' portion of the prochymosin gene fused to the signal sequence from the A. oryzae TAKA amylase gene is used as the starting material. A BglII restriction site is introduced immediately upstream of the ATG start codon of the signal sequence by polymerase chain reaction (PCR) (R.K. Saiki et al., Science 239, 1988, pp. 487-491) using the Gene Amp kit as recommended by the manufacturer (Perkin-Elmer). The following synthetic oligodeoxyribonucleotides are used primers for the reaction:
I: GAAGATCTGAAGGCATTTATGATGGTC
II: CCCAAAGTACTGACTATC
100 »l reaction mixture contains 25 pmol of each primer and 0.01 »g of pToC51. The approximately 290 bp PCR product is cleaved with the restriction enzymes BglII and PstI, and a 190 bp fragment is isolated on an agarose gel. This fragment is cloned into BglII/PstI digested pIC19R (described in J.L. Marsh et al., Gene 32, 1984, pp. 481-485). Plasmid DNA is recovered from the resulting transformants, and the insert is sequenced according to the method of Sanger (cf. Sanger et al., Proc. Natl. Acad. Sci. USA 74, 1977, pp. 5463-5467). The fragment comprises a DNA sequence coding for the TAKA amylase signal sequence and the 5' portion of the prochymosin gene.

The 190 bp BGlII-PstI fragment was then excised from pIC19R, and ligated with an approximately 350 bp PstI-XmaI fragment of pToC56 (described in Example 12 of EP 238023) containing part of the prochymosin coding sequence, and an approximately 1.4 kb XmaI-ClaI fragment of pToC56 containing the 3' half of the prochymosin gene and the A. niger glucoamylase gene transcription terminator and an approximately 3.7 kb BglIII ClaI fragment of p408.3 (described in Example 6 of EP 238023) containing the A. niger glucoamylase promoter cloned into the plasmid pIC19R.

After ligation, the ligation mixture was transformed into competent E. coli MC1000 (M.J. Casadaban and S.N. Cohen, J.Mol.Biol. 138, pp. 179-209) cells. Transformants were selected for ampicillin resistance and the plasmid construction verified by several restriction enzyme digestions analysed on agarose gels. The resulting plasmid pToC195 thus contains the A. niger glucoamylase promoter followed by the A. oryzae TAKA-amylase signal sequence fused in frame to the coding sequence of prochymosin and the transcription terminator from the A. niger glucoamylase gene.

### EXAMPLE 2

### Expression of prochymosin from the TAKA amylase signal sequence

The expression vector pToC195 was co-transformed into A. niger by the general procedure described in Example 9 of EP 238 023, using the plasmid p3SR2 which contains the A. nidulans amdS gene as the selection marker. After two reisolations of conidiospores, transformants were fermented in 10 ml of YPD medium (Sherman et al., Methods in Yeast Genetics, Cold Spring Harbor, 1981). The content of prochymosin in the fermentation broth was determined by ELISA on a Western blot after SDS-PAGE, using an antibody raised against chymosin. The transformant producing the highest yield (about 100 mg/l)of prochymosin of the correct size (as determined by Western blot) was then fermented in a 2 litre Kieler fermentor in a synthetic medium containing maltodextrin and urea. The fermentation is a batch fermentation where the pH is kept at 6 by means of NaOH. The chymosin content in the supernatant is determined by Western blotting. Several smaller immunoreactive bands are detected. These were presumed to be degradation products of prochymosin, i.e. the prochymosin was partly subjected either to automaturation or to degradation by A. niger proteases present in the fermentation broth.

### REFERENCE EXAMPLE

### A. Construction of the prochymosin expression plasmid pMT837

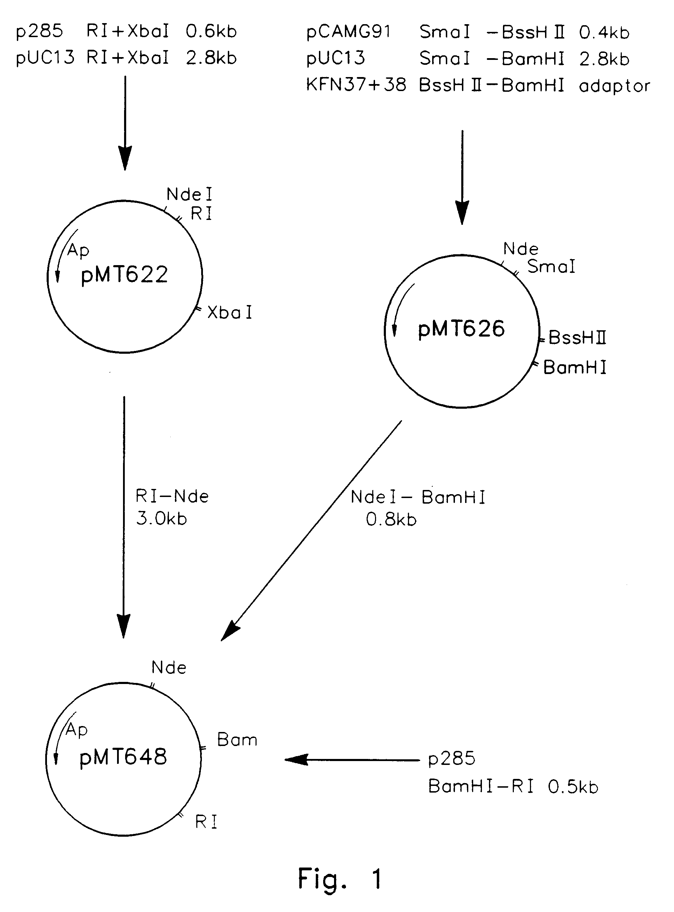

The construction of pMT837 is illustrated in Figs. 1-3.

The sequence encoding the C-terminal part of chymosin was isolated as a 0.6 kb EcoRI-Xba1 fragment and subcloned into pUC13 to give pMT622 (Fig. 1). Likewise a fragment from the AMG promoter region including the sequence encoding the glucoamylase prepropeptide was isolated from pCAMG91. This Sma1-BssH2 fragment of approximately 0.4 Kb was cloned into Sma1-BamH1 cut pUC13 together with the BssH2-BamH1 adaptor/linker KFN37 and 38 (Fig. 1). The sequence of KFN37 is: CGCGCTGAGATCACCAG and of KFN38: GATCCTGGTGATCTCAG. The linker serves to obtain a sequence encoding a perfect fusion from the glucoamylase prepropeptide into prochymosin. The 3.0 kb EcoR1-Nde1 fragment from pMT622 was ligated to the 0.8 kb Nde1-BamH1 fragment from pMT626 and the 0.5 kb BamH1-EcoR1 fragment from p285 (Fig. 1) to give pMT648. pMT648 therefore contains a short AMG promoter fragment in front of the sequence encoding the glucoamylase prepropeptide fused to the entire prochymosin.

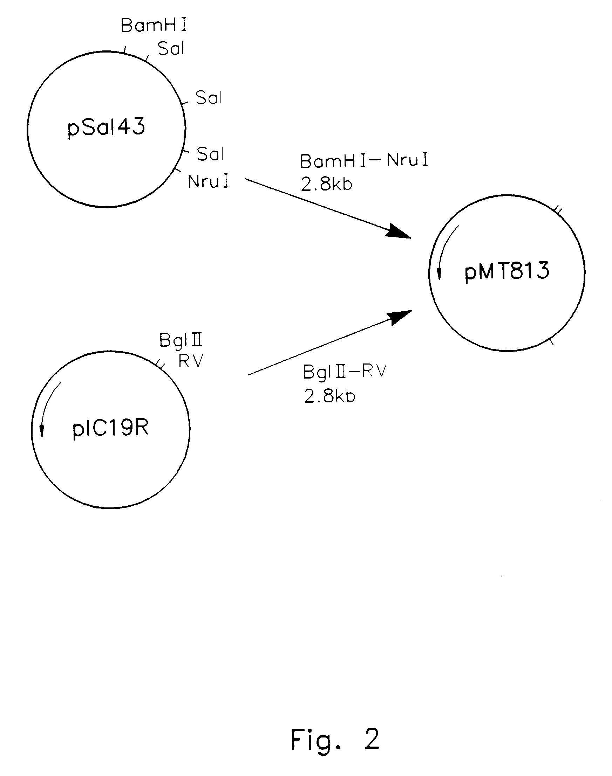

The argB gene of A. nidulans was subcloned from pSa143 (Gene 25 (1983) 109-117) as a 2.8 kb BamH1-Nru1 into Bgl2-EcoRV cut plC19R to give pMT813 (Fig. 2).

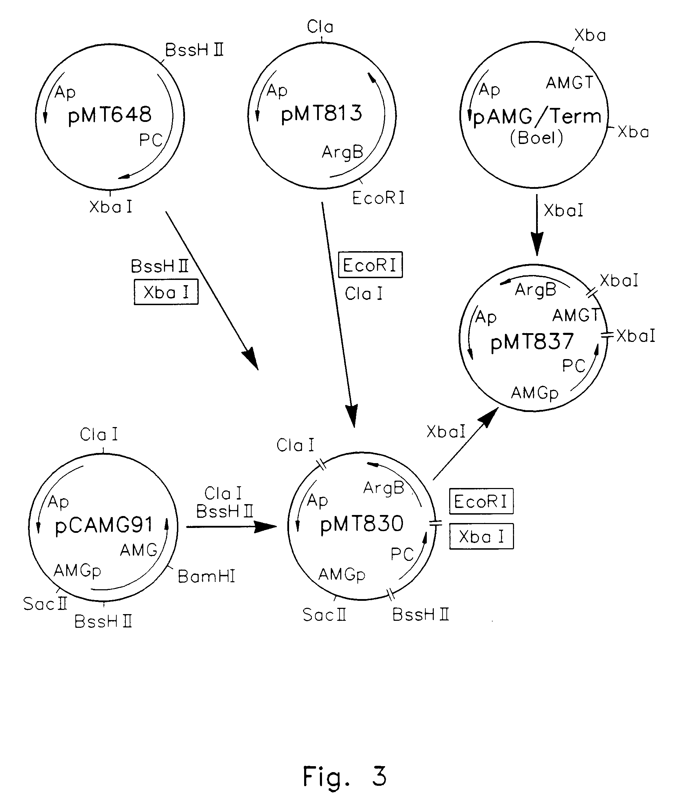

In order to include the argB marker in the expression plasmid and also to introduce a longer AMG promoter fragment the following steps were undertaken (Fig. 3), pMT648 was cut with Xba1 and blunt ended by Klenow polymerase and dNTP's, subsequently cut with BssH2 and a 1.1 kb fragment isolated. This fragment contains the prochymosin encoding sequence. Secondly, the argB gene was isolated from pMT813 as 2.8 kb Cla1-blunted EcoR1 fragment (EcoR1 filled in with Klenow and dNTP's). Finally a fragment containing the long AMG promoter was isolated as a 3.7 b Cla1-BssH2 fragment from pCAMG91. These three fragments were ligated to give pMT830 (Fig. 3). In a final step, pMT830 was cut with Xba1 (regenerated by the ligation and blunted EcoR1 and Xba1) dephosphorylated by alkaline phosphatase and ligated to an approximately 0.6 kb Xba1-Xba1 fragment from pAMG/Term containing the AMG transcription terminator (Fig. 3). The correct orientation of the terminator fragment was verified in the resulting plasmid pMT837.

### B. Expression of phochymosin from the AMG signal sequence

pMT837 was transformed into A. niger argB⁻ by the general procedure described in Example 9 of EP 238 023 using selection for argB present on the plasmid. Transformants were reisolated twice through conidiospores. The transformants were fermented in 10 ml of YPD medium. The content of chymosin in the fermentation broth was determined by ELISA on a Western blot after SDS-PAGE using an antibody raised against chymosin. The best transformant produced approximately 0.5 mg/l of immunoreactive material.

## Claims

1. A process for the production of chymosin or a derivative thereof having same properties in Aspergillus niger, the process comprising
(a) transforming an Aspergillus niger host organism with a recombinant DNA vector which comprises a DNA sequence encoding prochymosin or a derivative thereof having same properties N-terminally fused to a DNA sequence encoding a signal peptide derived from the Aspergillus oryzae TAKA amylase gene or a derivative of said signal peptide having same properties,
(b) culturing the transformed filamentous fungus host organism in a suitable culture medium under conditions conducive to the expression of prochymosin and secretion thereof to the medium, and
(c) recovering the prochymosin or chymosin or derivative thereof from the medium.

2. A process according to claim 1, wherein the DNA vector further comprises a promoter, selected from the group consisting of the A. niger glucoamylase promoter and the A. oryzae TAKA amylase promoter.

## Patentansprüche

1. Verfahren zur Herstellung von Chymosin oder eines Derivats davon mit den gleichen Eigenschaften in *Aspergillus niger*, wobei das Verfahren folgende Stufen umfaßt:
(a) Transformation von *Aspergillus niger* mit einem rekombinanten DNA-Vektor, der eine DNA-Sequenz umfaßt, die für Prochymosin oder ein Derivat davon mit den gleichen Eigenschaften codiert und N-terminal an eine DNA-Sequenz, die für ein Signalpeptid, das aus dem *Aspergillus oryzae*-TAKA-Amylasegen abgeleitet ist, oder ein Derivat des Signalpeptids mit den gleichen Eigenschaften codiert, fusioniert ist;
(b) Züchten des transformierten filamentösen Pilz-Wirtsorganismus in einem geeigneten Kulturmedium unter Bedingungen, die die Expression von Prochymosin und dessen Absonderung in das Medium fördern; und
(c) Gewinnung von Prochymosin oder Chymosin oder eines Derivates davon aus dem Medium.

2. Verfahren nach Anspruch 1, wobei der DNA-Vektor ferner einen Promotor umfaßt, der aus der Gruppe ausgewählt ist, die aus dem *A. niger*-Glucoamylase-Promotor und dem *A. oryzae*-TAKA-Amylase-Promotor besteht.

## Revendications

1. Procédé de préparation de chymosine ou d'un dérivé de celle-ci ayant les mêmes propriétés dans Aspergillus niger, le procédé consistant à
(a) transformer un organisme hôte d'Aspergillus niger avec un vecteur d'ADN recombinant qui comporte une séquence d'ADN codant pour la prochymosine ou pour un dérivé de celle-ci ayant les mêmes propriétés en ce qui concerne l'extrémité N-terminale fusionnée à une séquence d'ADN codant pour un peptide signal dérivé du gène de l'amylase TAKA d'Aspergillus oryzae ou pour un dérivé dudit peptide signal ayant les mêmes propriétés,
(b) cultiver l'organisme hôte de champignon filamenteux transformé dans un milieu de culture approprié dans des conditions conduisant à l'expression de la prochymosine et à la sécrétion de celle-ci dans le milieu, et
(c) récupérer la prochymosine ou la chymosine ou un dérivé de celles-ci à partir du milieu.

2. Procédé selon la revendication 1, dans lequel le vecteur d'ADN comporte de plus un promoteur, choisi parmi le groupe composé du promoteur de la glucoamylase d'A. niger et du promoteur de l'amylase TAKA d'A. oryzae.
